# EUROPEAN PATENT APPLICATION

(11) **EP 4 261 542 A1**
(43) Date of publication of application: **18.10.2023**
(21) Application number: 22167551.5
(22) Date of filing: 11.04.2022
(51) Int. Cl.: G01N 33/82, G01N 33/543

(54) **METHOD AND DEVICE FOR DETERMINING THE VITAMIN STATUS**

(71) Applicant: Technische Universität München, 80333 München (DE)
(72) Inventor: Sieber, Stephan, 86919 Utting (DE); Bach, Nina, 85457 Wörth (DE); Fiedler, Michaela, 83324 Ruhpolding (DE); Drechsel, Jonas, 80805 München (DE)
(74) Representative: Engelhard, Markus

(57) **Abstract**

The present invention relates to *in vitro* methods for determining the vitamin status of a subject, in particular health related vitamins, such as B2, B6, B12 and/or D3. The present invention further relates to a kit and an immunographic device for *in vitro* determining the vitamin status of a subject.

## Description

The present invention relates to *in vitro* methods for determining the vitamin status of a subject, in particular health related vitamins, such as B2, B6, B12 and/or D3. The present invention further relates to a kit and an immunographic device for *in vitro* determining the vitamin status of a subject.

### BACKGROUND OF THE INVENTION

Vitamin B6 refers to a collection of vitamers including the enzyme cofactor pyridoxal phosphate (PLP). B6 is an essential nutrient that has to be taken up by a well-balanced diet. While the supply of B6 in wholesome foods including meat, bananas and potatoes is sufficient, processing of meals can significantly reduce the amount of B6 vitamers due to their limited stability (Gregory and Kirk, 1977).

The Centers for Disease Control and Prevention showed in a study that about 10 % of the US population exhibits plasma PLP levels which are below 20 nmol/L, considered as threshold value for sufficient supply, indicating an inadequate B6 status (Center for Disease Control and Prevention, 2012). Malnutrition, as often observed in developing countries, further intensifies the problem. B6 deficiency is associated with various symptoms including higher rates of cardiovascular disease, stroke and cancer (Verhoef *et al.,* 1996; Robinson *et al*., 1998; Sies *et al.,* 1992; Vanuzzo *et al.,* 2007; Dalery *et al.,* 1995; Rimm *et al.,* 1998; Johansson *et al.,* 2010; Kelly *et al.,* 2004; Lotto *et al.,* 2011). Populations at risk to exhibit critically low B6 levels include elderly people, diabetes patients and alcoholics. Additionally, healthy people with increased needs of B6, such as pregnant and lactating women require a stringent and continuous monitoring of B6 levels. Here, several diagnostic procedures are on the market. Plasma PLP is the most prevalent biomarker used (Leklem, 1990), which is detected by high performance liquid chromatography (HPLC) as part of laboratory diagnostics. Major challenges in its determination are the
- overall stability of PLP, whose plasma concentration declines in samples kept at room temperature and exposed to light (Hustad *et al.,* 2012; Panton *et al.,*2013; van der Ham *et al*., 2012);
- inflammation, albumin binding, alkaline phosphatase activity and alcohol consumption can result in a 30 - 40 % variance (Brussaard *et al.,* 1997; Ueland *et al.,* 2015).

Thus, erythrocyte PLP determination via HPLC or functional assays have been identified as a more relevant marker of the B6 status also reflecting the fact, that the vitamer is an intracellular cofactor (Leklem, 1990; Ueland *et al.,* 2015; Vermaak *et al*., 1990). Blood can be rapidly drawn from patients providing an opportunity to directly monitor effects of B6 supplementation. However, a disadvantage of direct PLP measurement is the binding of PLP to hemoglobin, lowering the overall precision (Ueland *et al.,* 2015; Reynolds *et al.,* 1995). As an alternative, functional B6 biomarker tests are based on PLP-dependent enzymes (PLP-DEs) that are supposed to overcome these problems. Here, the transaminase test is one of the most mature assay systems monitoring the activity of aspartic acid transaminase or alanine transaminase in erythrocyte extract (Ueland *et al.,* 2015). While this assay overcomes above mentioned limitations of free-PLP detection, a major challenge is the time-consuming processing of fresh blood for these assays limiting the overall stability of PLP-DEs (Ueland *et al.,* 2015; Huang *et al.,* 1998).

As of yet, there is no diagnostic B6 test available which combines analysis of blood samples, detection of most relevant enzyme based-PLP-cofactor status and speed for a reliable analysis. No personalized parameters which are based on recording the status of multiple enzymes can be obtained *via* the methods of the prior art.

There is a need in the art for improved methods and means for determining the vitamin status of a subject.

### SUMMARY OF THE INVENTION

According to the present invention this object is solved by an *in vitro* method for determining the vitamin status of a subject comprising
(a) harvesting erythrocytes from a sample obtained from a subject or providing erythrocytes obtained from a sample of a subject;
(b) providing a probe comprising at least one functionalized cofactor of PLP-dependent enzymes (PLP-DEs) (PLP probe);
(c) lysing the erythrocytes and thereby releasing the proteome including the PLP-DEs and then treating the lysate with the PLP probe provided in (b); or
(c') treating the erythrocytes with the PLP probe provided in (b) and then lysing the erythrocytes and thereby releasing the proteome including the PLP-DEs;
(d) adding a label for the PLP-probe to the treated lysate of (c) or to the lysate of (c');
(e) capturing the labeled PLP-DEs with anti-PLP-DE-antibodies immobilized on a surface, preferably an array or a membrane,
(f) detecting the labeled PLP-DEs, and
(g) determining the vitamin status.

According to the present invention this object is solved by a kit for *in vitro* the vitamin status, in particular vitamin B6, B2, B12 and/or D3, in a sample of a subject, comprising
(1) a probe comprising at least one functionalized cofactor of PLP-dependent enzymes (PLP-DEs) (PLP probe);
(2) a label for the PLP-probe;
(3) optionally, an array for PLP-DEs, preferably comprising anti-PLP-DE-antibodies,
(4) optionally, reagents for lysing erythrocytes, reducing agents, and control(s).

According to the present invention this object is solved by an immunographic device for *in vitro* determining the vitamin status, in particular vitamin B6, B2, B12 and/or D3, in a sample of a subject,
said device comprising a solid carrier or surface coated with anti-PLP-DE-antibodies, wherein the immunographic device is preferably a lateral flow assay (LFA) device.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

### Method for determining the vitamin status

As outlined above, the present invention provides an *in vitro* method for determining the vitamin status of a subject.

Preferably, the vitamin status of health related vitamins is determined, in particular vitamin B2, B6, B12 and/or D3.

Said method comprises the steps of:
(a) harvesting erythrocytes from a sample obtained from a subject or providing erythrocytes obtained from a sample of a subject;
(b) providing a probe comprising at least one functionalized cofactor of PLP-dependent enzymes (PLP-DEs) (PLP probe);
(c) lysing the erythrocytes and thereby releasing the proteome including the PLP-DEs and then treating the lysate with the PLP probe provided in (b);
   or
(c') treating the erythrocytes with the PLP probe provided in (b) and then lysing the erythrocytes and thereby releasing the proteome including the PLP-DEs;
(d) adding a label for the PLP-probe to the treated lysate of (c) or to the lysate of (c');
(e) capturing the labeled PLP-DEs with anti-PLP-DE-antibodies immobilized on a surface, preferably an array or a membrane,
(f) detecting the labeled PLP-DEs, and
(g) determining the vitamin status.

In one embodiment, the method comprises the steps of:
(a) harvesting erythrocytes from a sample obtained from a subject or providing erythrocytes obtained from a sample of a subject;
(b) providing a probe comprising at least one functionalized cofactor of PLP-dependent enzymes (PLP-DEs) (PLP probe);
(c) lysing the erythrocytes and thereby releasing the proteome including the PLP-DEs and then treating the lysate with the PLP probe provided in (b);
(d) adding a label for the PLP-probe to the treated lysate of (c);
(e) capturing the labeled PLP-DEs with anti-PLP-DE-antibodies immobilized on a surface, preferably an array or a membrane,
(f) detecting the labeled PLP-DEs, and
(g) determining the vitamin status.

In an alternative embodiment, the method comprises the steps of:
(a) harvesting erythrocytes from a sample obtained from a subject or providing erythrocytes obtained from a sample of a subject;
(b) providing a probe comprising at least one functionalized cofactor of PLP-dependent enzymes (PLP-DEs) (PLP probe);
(c') treating the erythrocytes with the PLP probe provided in (b) and then lysing the erythrocytes and thereby releasing the proteome including the PLP-DEs;
(d) adding a label for the PLP-probe to the lysate of (c');
(e) capturing the labeled PLP-DEs with anti-PLP-DE-antibodies immobilized on a surface, preferably an array or a membrane,
(f) detecting the labeled PLP-DEs, and
(g) determining the vitamin status.

### Step (a)

In step (a) of the method of the present invention, erythrocytes are harvested from a sample obtained from a subject or erythrocytes obtained from a sample of a subject are provided.

In the embodiment, where the erythrocytes are harvested from a sample, they are preferably harvested by centrifugation, optionally including washing step(s).

The sample is preferably whole blood.

### Step (b)

In step (b) of the method of the present invention, a probe is provided which comprises at least one functionalized cofactor of PLP-dependent enzymes (PLP-DEs) (PLP probe).

PLP-DEs have been implicated in human disease and are recognized as important drug targets. PLP-DEs include aspartic acid transaminase, alanine racemase, serine hydroxymethyltransferase, ornithine decarboxylase, GABA aminotransferase, DOPA decarboxylase, and branched-chain amino acid aminotransferase. PLP acts as a coenzyme in all transamination reactions, and in certain decarboxylation, deamination, and racemization reactions of amino acids. PLP is also involved in various beta-elimination reactions such as the reactions carried out by serine dehydratase and GDP-4-keto-6-deoxymannose-3-dehydratase (ColD).

Preferably, the cofactor(s) of PLP-dependent enzymes (PLP-DEs) are functionalized with a biorthogonal labeling group.

In one embodiment, the cofactor(s) of PLP-dependent enzymes (PLP-DEs) are functionalized with a biorthogonal labeling group and optionally further with a phosphoamidate capping group.

The term "bioorthogonal chemistry" refers to any chemical reactions that can occur inside of living systems or in biological samples in general, that do not interfere with the biological system as well as side reactions in the organism or with the biological sample do not take place making bioorthogonal chemistry extremely selective reactions within complex biological matrices.

A number of chemical ligation strategies have been developed that fulfill the requirements of bioorthogonality, including
1,3-dipolar cycloaddition between azides and cyclooctynes (also termed copper-free click chemistry), between nitrones and cyclooctynes, oxime/hydrazone formation from aldehydes and ketones, the tetrazine ligation, the isocyanide-based click reaction, and the quadricyclane ligation. Furthermore the copper catalyzed azide alkyne click reaction (CuAAC) and the Staudinger ligation.

The use of bioorthogonal chemistry usually proceeds in two steps. First, a (cellular) substrate is modified with a bioorthogonal functional group ("biorthogonal labeling group"). Secondly, a probe containing the complementary functional group is introduced to react and label the substrate.

In a preferred embodiment, the biorthogonal labeling group is used to modify or functionalize the cofactor(s) of the PLP-DEs. The complementary functional group is added to the label.

The biorthogonal labeling group is preferably selected from an alkyne or azide moiety or a tetrazine functionality.

In a preferred embodiment, the biorthogonal labeling group is an alkyne group.

The alkyne group can be attached directly to the pyridine ring of the cofactor (such as PL1 or PL1P) or with a spacer, such as an ethylene spacer (such as PL2 or PL2P).

The phosphorylated probes (PLxP) can be obtained from the reaction of the respective PLx probe with the enzyme *S. Aureus* protein kinase PLK, such as described in Hoegl *et al.* (2018) or Fux *et al.* (2019).

The functionalized cofactors are preferably selected from PL1P, PL2P, PL4P to PL13P.
PL1P : (6-ethynyl-4-formyl-5-hydroxypyridin-3-yl)methyl phosphate
PL2P : (4-formyl-6-(hept-6-yn-1-yl)-5-hydroxypyridin-3-yl)methyl phosphate
PL4P : (6-(but-3-yn-1-yl)-4-formyl-5-hydroxypyridin-3-yl)methyl phosphate
PL5P : (6-(4-ethynylphenethyl)-4-formyl-5-hydroxypyridin-3-yl)methyl phosphate
PL6P : (4-formyl-5-hydroxy-6-(pent-4-yn-1-yl)pyridin-3-yl)methyl phosphate
PL7P : (4-formyl-6-(hex-5-yn-1-yl)-5-hydroxypyridin-3-yl)methyl phosphate
PL8P : (4-formyl-5-hydroxy-6-(2-(1-(prop-2-yn-1-yl)-1H-1,2,3-triazol-4-yl)ethyl)pyridin-3-yl)methyl phosphate
PL9P : (6-(2-(1-(but-3-yn-1-yl)-1H-1,2,3-triazol-4-yl)ethyl)-4-formyl-5-hydroxypyridin-3-yl)methyl phosphate
PL10P : (2-ethynyl-4-formyl-5-hydroxy-6-methylpyridin-3-yl)methyl phosphate
PL11P: (4-formyl-5-hydroxy-6-methyl-2-(1-(prop-2-yn-1-yl)-1H-1,2,3-triazol-4-yl)pyridin-3-yl)methyl phosphate
PL12P : (2-(but-3-yn-1-yl)-4-formyl-5-hydroxy-6-methylpyridin-3-yl)methyl phosphate PL13P : (4-formyl-6-methyl-5-(prop-2-yn-1-yloxy)pyridin-3-yl)methyl phosphate,

In a preferred embodiment, the biorthogonal labeling group is an azide group, and the cofactor is preferably PL3P.

PL3P: (6-(azidomethyl)-4-formyl-5-hydroxypyridin-3-yl)methyl phosphate

In a preferred embodiment, the cofactor of PLP-DEs is further functionalized with a phosphoamidate.

In one embodiment, the probe comprises at least two functionalized cofactors of PLP-DEs or it comprises three or more functionalized cofactors.

In one embodiment, the probe comprises a further moiety or tag or further moieties/tags, such as biotin.

### Step (c)

In step (c) of the method of the present invention, the erythrocytes are lysed and thereby release the proteome including the PLP-DEs.

Preferably, the lysis of the erythrocytes comprises the removal of hemoglobin.

Removal of hemoglobin is preferred, because PLP binds to hemoglobin, which can disturb the result of the method of the present invention.

In one embodiment, step (c) comprises the addition of a lysis buffer, preferably in a ratio erythrocytes:buffer = 1:1. The lysis buffer can be any suitable buffer to lyse the cells, such as Tris buffer (Tris 50 mM, NaCl 150 mM, 1% NP40 (v/v), pH=7.5). Step (c) can further comprise incubation with lysis buffer (preferably on ice) for 5 min and removal of hemoglobin with NiNTA-agarose beads (e.g. ratio ~ 10 µL lysate to 100 µL beads).

Step (c) of the method of the present invention further comprises that the lysate obtained is treated with the PLP probe provided in (b).

Preferably, the treatment comprises an incubation of the lysate with the PLP probe and optional addition of a reducing agent.

Under reducing conditions, the PLP probe will bind irreversibly to the PLP DEs.

In one embodiment, reduction can be performed - if necessary - with sodium borohydride NaBH₄.

### Step (c')

In step (c') of the method of the present invention, the erythrocytes are treated with the PLP probe provided in (b).

Said treatment with the PLP probe is carried out before lysing the erythrocytes.

Preferably, the PLP probe is a cofactor of PLP-DEs which is (further) functionalized with a phosphoamidate.

Preferably, the treatment comprises an incubation of the lysate with the PLP probe and optional addition of a reducing agent.

In step (c'), after the treatment, the erythrocytes are lysed and thereby release the proteome including the PLP-DEs.

Preferably, the lysis of the erythrocytes comprises the removal of hemoglobin.

Removal of hemoglobin is preferred, because PLP binds to hemoglobin, which can disturb the result of the method of the present invention.

In one embodiment, step (c') comprises the addition of a lysis buffer, preferably in a ratio erythrocytes:buffer = 1:1. The lysis buffer can be any suitable buffer to lyse the cells, such as Tris buffer (Tris 50 mM, NaCl 150 mM, 1% NP40 (v/v), pH=7.5). Step (c) can further comprise incubation with lysis buffer (preferably on ice) for 5 min and removal of hemoglobin with NiNTA-agarose beads (e.g. ratio ~ 10 µL lysate to 100 µL beads).

### Step (d)

In step (d) of the method of the present invention, a label for the PLP-probe is added to the treated lysate of (c) or the lysate of (c').

Preferably, the label in step (d) is
a fluorescent label,
   such as rhodamines, cyanine dyes (Cy3, Cy5), fluoresceines,
a bioluminescence or chemiluminescence label,
   such as isoluminol and its derivatives, luciferins,
a dye,
   or combinations thereof.

The label is preferably attached to the biorthogonal labeling group of the cofactor(s).

The label preferably comprises a complementary functional group to the biorthogonal labeling group of the cofactor(s). Both the biorthogonal labeling group and the respective complementary functional group react with each other, such as via click chemistry or Staudinger ligation.

### Step (e)

In step (e) of the method of the present invention, the labeled PLP-DEs are captured with anti-PLP-DE-antibodies immobilized on a surface.

The surface is preferably an array or a membrane. Suitable surfaces are known to the skilled artisan.

The anti-PLP-DE-antibodies can be monoclonal or polyclonal antibodies. Such antibodies are commercially available. For example:
Anti PLPHP_1 Thermofischer, cat. nr. PA532036, polyclonal
Anti PLPBP_2 Novus Biologicals, cat. No. 21730002 polyclonal
Anti PLPBP _3 Origene, cat. nr. TA505130, monoclonal
Anti OAT_1 Origene, cat. AM09363PU-S, polyclonal
Anti OAT_2 Abcam, cat. Ab137679, polyclonal
Anti OAT_3 Novus Biologicals, cat. NBP1-49312, monoclonal
Anti SHMT1_1 Abcam, cat. Ab186130, polyclonal
Anti SHMT1_2 Merck, cat. ABE416, polyclonal
Anti SHMT1_3 Novus Biologicals, cat. NBP2-32173, polyclonal

In a preferred embodiment, in or after step (e) a second anti-PLP-DE-antibody is added.

Said second anti-PLP-DE-antibody is not immobilized on a surface. Said second anti-PLP-DE-antibody comprises a label, which is different from the label of the PL-P-probe.

Preferably, the label of the second anti-PLP-DE-antibody is
a fluorescent label,
   such as rhodamines, cyanine dyes (Cy3, Cy5), fluoresceines,
a bioluminescence or chemiluminescence label,
   such as isoluminol and its derivatives, luciferins,
a dye,
   or combinations thereof.

For example, the second anti-PLP-DE-antibody is a polyclonal antibody which can be the same as the capturing anti-PLP-DE-antibody due to its polyclonality.

### Step (f)

In step (f) of the method of the present invention, the labeled PLP-DEs are detected.

The method of detection and the readout depend on the label used.

In an embodiment, where the label is a fluorescent label, the fluorescence is detected and preferably fluorescence intensity is determined. The fluorescence intensity can be the readout. In an embodiment, where the label is a bioluminescence label, the bioluminescence is detected and preferably bioluminescence intensity is determined. The bioluminescence intensity can be the readout.

In an embodiment, where the label is a dye, the color can be detected or absorbance can be detected.

In an embodiment, where second anti-PLP-DE-antibodies are used, the label of said second antibody is detected as well.

In this embodiment, two signals are detected, where (1) the PLP-probe signal reflects the loading state of the cofactor (wherein higher PLP probe signal corresponds to a lower loading state of the cofactor) and (2) the antibody signal reflects the total amount of PLP-DE enzyme in the sample. By comparing these to signals, a ratio PLP loading state/total PLP-DE amount is obtained.

Healthy patients exhibit roughly the same ratio, whereas vitamin B6 compromised patients exihibit higher ratios.

For example, in case of vitamin B6 deficiency, little PLP is bound to PLP-DE, such that more PLxP probe can bind to the PLP-DEs which results in a higher signal.

Additionally, the free vitamin b6 status of the patients in blood can be determined via HPLC to calibrate the ratios.

### Step (g)

In step (g) of the method of the present invention, the vitamin status is determined.

Preferably, the vitamin status is determined by comparing the readout of step (f), such as fluorescence intensity, bioluminescence intensity, or absorbance, with a control.

Preferred controls are recombinant PLP-DEs with defined cofactor loading or reference samples where the PLP status is known, e.g. in form of a calibration curve.

Preferably, the vitamin B6 status of the subject is determined.

In a preferred embodiment, the method of the present invention is a point of care method.

Point-of-care testing (POCT) is a form of testing in which the analysis is performed where healthcare is provided close to or near the patient. POCT can be undertaken in many locations including: home use and self testing.

In preferred embodiments, the method of the present invention further comprises the steps of:
dividing the sample obtained from the subject in two parts and pre-treating one part with a PLP standard prior to step (c),
performing steps (a) to (f) for both parts in parallel,
comparing the results of step (f) for both parts and thereby quantifying the PLP status.

Preferably, the vitamin status determined in a subject is used for determining the vitamin B6 status of the subject.

Preferably, the vitamin status determined in a subject is used for monitoring the PLP status and/or vitamin B6 status of the subject.

Preferably, the vitamin status determined in a subject is used for diagnosing vitamin B6-deficiency and vitamin B6-deficiency-associated diseases,
in particular related to age, malnutrition, and/or alcoholism.
in dialysis patients,
inflammatory bowel disease.

Vitamin B6 comprises six compounds based on a 2-methyl-3-hydroxypyridine structure with differing subunits at positions C4 and C5 that are interconvertible. These are pyridoxine, pyridoxamine, and pyridoxal and their phosphorylated derivatives pyridoxine-5-phosphate, pyridoxamine-5-phosphate, and pyridoxal-5-phosphate (PLP). PLP is a cofactor in > 150 different enzyme reactions including transamination and decarboxylation enzyme systems. The symptoms of vitamin B₆ deficiency include peripheral neuropathy, pellagra-like syndrome with seborrheic dermatitis and glossitis. Prolonged deficiency can lead to depression, confusion, and in severe cases causes abnormalities in EEG signals and seizures. Vitamin B₆ deficiency is often associated with deficiencies in other B vitamins and can be due to overall poor nutrition caused by a chaotic lifestyle, e.g., in alcoholic subjects. Suboptimal vitamin B₆ status has also been reported in oral contraceptive users, smokers, and patients with celiac disease or diabetes Secondary vitamin B₆ deficiency can occur in treatments with drugs that interact with PLP, e.g., isoniazid or inborn errors in vitamin B₆ salvage pathways or when mutations result in the accumulation of intermediates that react with PLP. Vitamin B₆ deficiency has been reported to be associated with an increased risk of cardiovascular disease, stroke, and cancer. Low plasma PLP has been reported in a number of diseases associated with inflammation including rheumatoid arthritis, inflammatory bowel disease, diabetes, and deep vein thrombosis.

### Test device and kit

As outlined above, the present invention provides a kit for *in vitro* determining the vitamin status in a sample of a subject.

Preferably, the vitamin status of health related vitamins is determined, in particular vitamin B2, B6, B12 and/or D3.

Said kit comprises
(1) a probe comprising at least one functionalized cofactor of PLP-dependent enzymes (PLP-DEs) (PLP probe);
(2) a label for the PLP-probe;
(3) optionally, an array for PLP-DEs, preferably comprising anti-PLP-DE-antibodies,
(4) optionally, reagents for lysing erythrocytes, reducing agents, and control(s).

As outlined above, the present invention provides an immunographic device for *in vitro* determining the vitamin status in a sample of a subject.

Preferably, the probe comprising at least one functionalized cofactor of PLP-dependent enzymes (PLP-DEs) (PLP probe) is as defined herein above.

Preferably, the label for the PLP-probe is as defined herein above.

Preferably, the vitamin status of health related vitamins is determined, in particular vitamin B2, B6, B12 and/or D3.

Said device comprises a solid carrier or surface coated with anti-PLP-DE-antibodies or a solid carrier or surface coated with nanoparticles comprising a moiety or tag which binds to a probe comprising at least one functionalized cofactor of PLP-dependent enzymes (PLP-DEs) (PLP probe).

Preferably, the immunographic device is a lateral flow assay (LFA) device, which is more preferably a point-of-care device.

In an embodiment, the immunographic device comprises a porous membrane operably connected to
(a) a portion or pad, where a processed sample is applied,
(b) a test portion or test line comprising said anti-PLP-DE-antibodies or said nanoparticles, preferably gold nanoparticles,
(c) a control portion or test line; and
(d) an absorbent portion/pad.

Preferably, the sample is whole blood or erythrocytes.

In an embodiment, when the sample is whole blood, the erythrocytes are harvested therefrom, as decribed above

In a preferred embodiment, obtaining a processed sample, which is applied to the device of the present invention, comprises:
the erythrocytes or the harvested erythrocytes are lysed and thereby release the proteome including the PLP-dependent enzymes (PLP-DEs),
the lysate is treated with a probe comprising at least one functionalized cofactor of PLP-dependent enzymes (PLP-DEs) (PLP probe), said probe being as defined herein above, and
a label for the PLP-probe, which is as defined herein above, is added to the treated lysate, and
the processed sample is obtained.

In an alternative embodiment, obtaining a processed sample, which is applied to the device of the present invention, comprises:
the erythrocytes or the harvested erythrocytes are treated with a probe comprising at least one functionalized cofactor of PLP-dependent enzymes (PLP-DEs) (PLP probe), said probe being preferably a cofactor of PLP-DEs which is functionalized with a phosphoamidate,
the treated erythrocytes are then lysed and thereby release the proteome including the PLP-dependent enzymes (PLP-DEs),
a label for the PLP-probe, which is as defined herein above, is added to the lysate, and
the processed sample is obtained.

In an embodiment, the PLP-probe comprises a biotin tag and the gold nanoparticles in the test portion/line are streptavidin-gold nanoparticles.

Preferably, the control portion or control line comprises antibodies against streptavidin, or biotin or biotin derivatives to capture the streptavidin-gold nanoparticles.

### Preferred embodiments

The inventors developed chemical PLP probes which successfully captured 75 % of all known bacterial PLP-DEs. These probes largely mimic the pyridoxal core scaffold and bear a biorthogonal alkyne handle for click chemistry to a reporter tag. In a previous study, published under Hoegl A. et al. (Nature Chemistry, 2018), we showed that the probes infiltrate cognate pyridoxal uptake into bacteria, get phosphorylated to the corresponding PLP probes by pyridoxal kinases and finally become incorporated into PLP-DEs. The inventors also applied this methodology to study the human PLP-ome (published under Fux et al. (Cell Chemical Biology, 2019), where they demonstrated the utility of these PLP-cofactor mimics for the identification of human PLP-binding proteins in live cells.

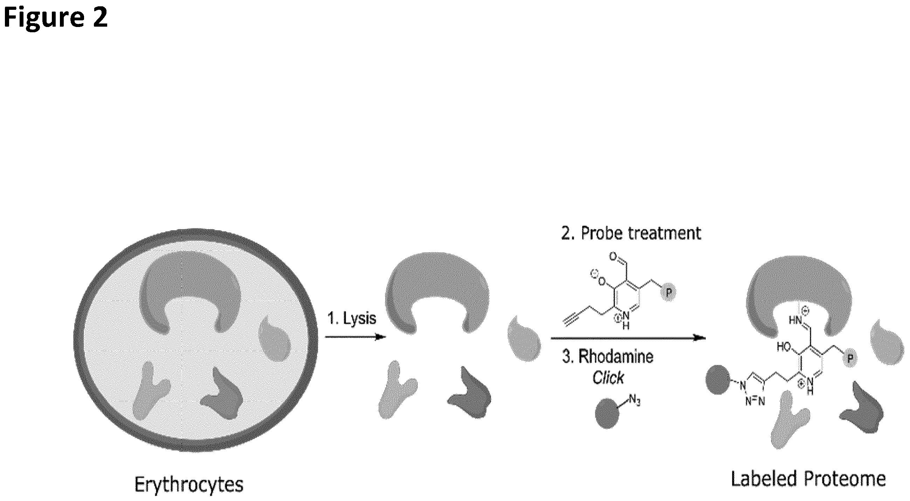

In the present application, it is first shown that PLP probes are highly versatile tools to directly determine the PLP status in human erythrocytes. See Figure 2.

Human erythrocytes express seven known PLP-dependent enzymes (Bryk *et al.,* 2017) and the activity of PLP-DEs is generally dependent on the amount of PLP bound (Ueland et al., 2015). The inventors show that a PLP probe can immediately monitor the PLP bound status of all seven enzymes in parallel without the need of performing individual activity assays. This not only enhances the reliability but also provides personalized parameters due to an assessment of multiple enzymes with essential cellular functions. For example, if the PLP status is low more probe binding will be observed to these enzymes and accordingly a higher probe-bound PLP-DE signal will be obtained. If the levels are high, the signal goes down. Such a direct assay provides a readout of the overall B6 status (direct parameter) and furthermore yields individual parameters on several signature PLP-DEs which may correlate with individual disease predispositions (personalized parameter).

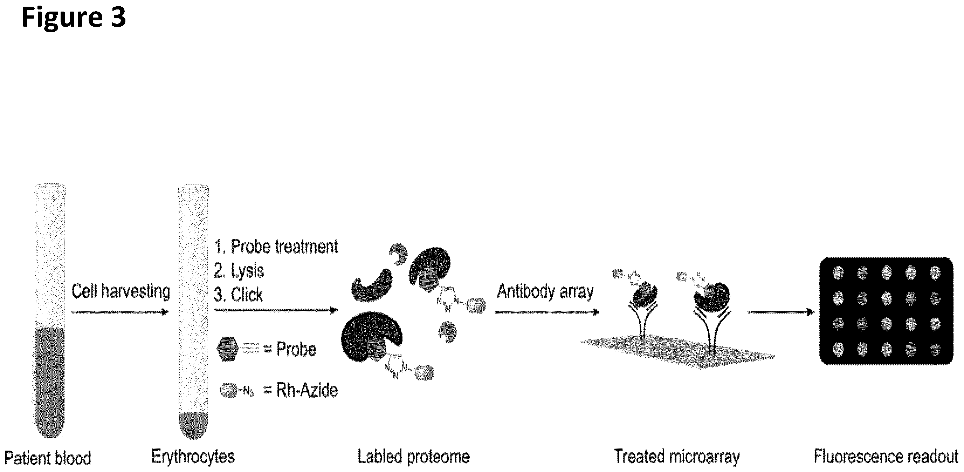

The invention provides a method as well as a ready-to-use device, termed B6VitaStat, which is able to directly monitor the PLP status of human erythrocytes based on multiple readout parameters. Erythrocytes are treated with our PLP cofactor mimics. Upon lysis of erythrocytes, PLP-DEs are labeled with our PLP probe and subsequently clicked to a fluorescent rhodamine tag. Labeled proteins are captured by commercial antibodies against PLP-DEs which are immobilized on a surface, such as glass slides (microarray) or membranes. The array is washed and analyzed in a fluorescent scanner. Recombinant PLP-DEs with defined cofactor loading are added to the lysate as internal standard to provide a quality control and facilitate a quantitative readout by comparison of signal intensities (Figure 3).

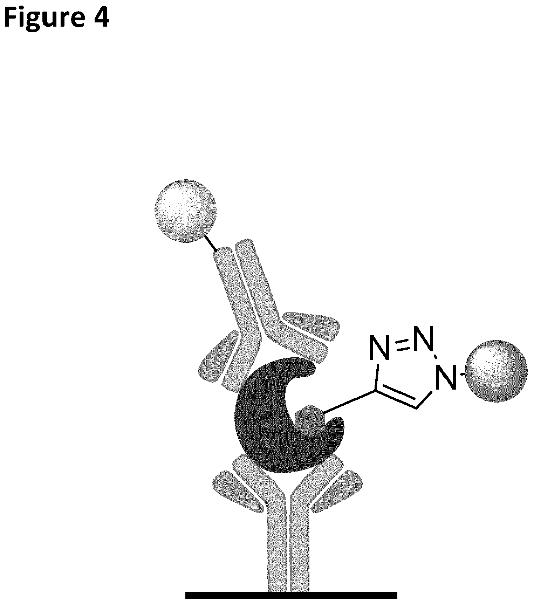

Alternatively for relative quantification, the patient's blood is split into two equivalent samples and one is pre-incubated with a defined PLP standard prior to probe treatment. Thus, less PLP probe can bind to the human PLP-DEs resulting in a lower (fluorescent) signal, whereas more probe is able to bind the non-pretreated sample leading to higher signals. Comparison of the obtained signals upon (fluorescent) readout on the microarray allows the determination of the patient's PLP status. Implementation of threshold values allows to facilitate the recognition of PLP deficiencies in humans (Figure 4).

Thus, the method of the present invention is able to determine the PLP status immediately, preserves the facile cofactor prior to degradation, provides personalized parameters based on multiple enzymes and is independent from unbound PLP in erythrocytes. Most importantly, this methodology does not require expensive HPLC-instruments and only a much cheaper fluorescent device is needed. Thus, the corresponding test can also be performed easily in developing countries, where malnutrition and B6 deficiency is a big problem.

Signal detection and amplification can also be adjusted to different settings including smartphone-based sensing methods (Kanchi *et al.,* 2018) paving the way for a ready-to-use device at family doctor offices or at home.

Our novel B6VitaStat platform is innovative as it provides a new approach to overcome limitations of established methods suffering from long processing times, indirect readouts, narrow scope and need for high-end instruments. Currently, tests comprise direct PLP determination *via* HPLC analysis or indirect PLP determination based on enzymatic activity (such as disclosed in German patent No. DE 000060037311 T2) or bioactivity (e.g. ID-Vit^{®}-Vitamin B6 Assay from Immundiagnostik, Germany). In contrast, B6VitaStat according to the present invention represents an improved diagnostic platform for continuous and direct monitoring of the vitamin B6 status directly from patient blood without the need of expensive equipment providing rapid processing analysis. This kind of monitoring is easy and can also be performed by nonmedical experts, e.g. at home or in developing countries. Like Vitamin D which can be detected by different immunoassay methods, such as electrochemiluminescent assay Elecsys^{®} (Roche Diagnostics) or Preventis SmarTest Pro^{®} Vitamin D, B6 determination can now be offered on a more frequent basis or even independent of diagnostic laboratories. For example, comparison of blood samples from different patients suffering under diverse B6-deficiency associated diseases will likely provide patterns characteristic for the respective pathologies. B6VitaStat thus not only provides rapid and cheap access to the nutritional B6 status but can also serve as a device linked to machine learning with important implications for personalized medicine and diagnostics.

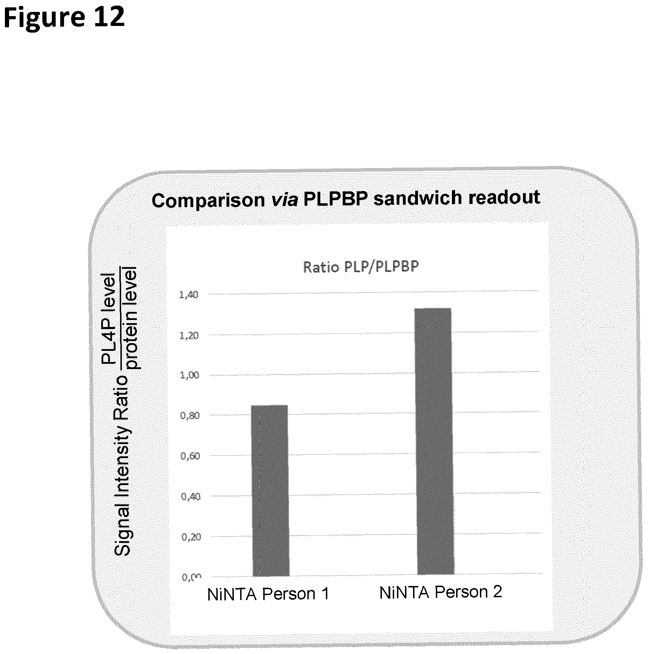

Furthermore, due to the dependency of the fluorescence intensity of the amount of protein present in the sample and the saturation state of the enzyme (i.e. the PLP loading status of PLP-DEs), the microarray can be extended to a sandwich format to enable normalization of signal intensities and to interpret the vitamin status of a subject. Therefore, after incubation of the PLP treated lysate on the microarrays, a second antibody bearing a different fluorescent label than the PLP probe is added and a two-channel fluorescence readout is performed. Herein, high ratios of PLP probe/protein amount indicate low vitamin B6 status. Comparison of two healthy volunteers with differences in their vitamin B6 status (as pre-determined via HPLC) verified this theory making this platform a novel platform for vitamin status assessment (see e.g. Figure 12).

The following examples and drawings illustrate the present invention without, however, limiting the same thereto.

### BRIEF DESCRIPTION OF THE DRAWINGS

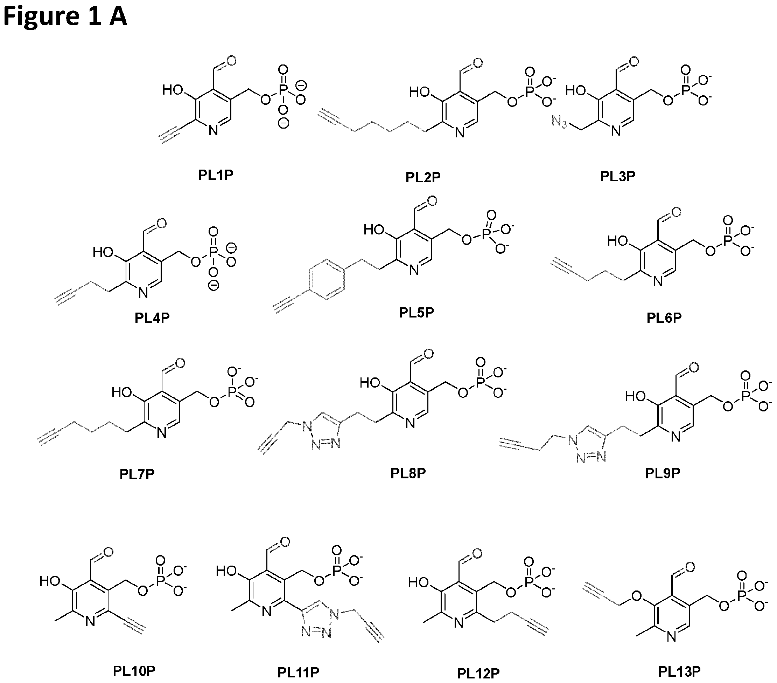

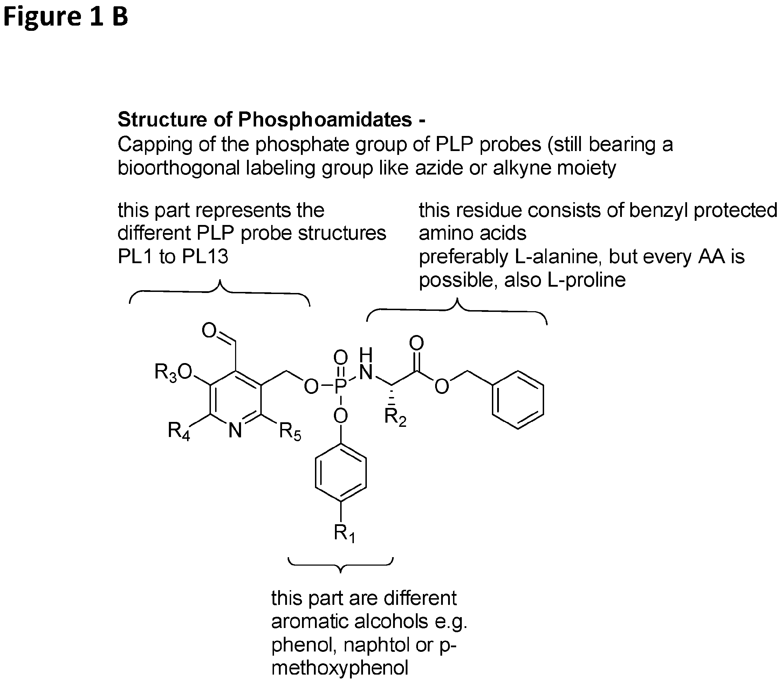

**Figure 1****.** *PLP probes.*
   **A,** Chemical structures of phosphorylated PLP probes (PLxP).
   **B,** Exemplary structure of PLP probes with a further phosphoamidate group.
**Figure 2****.** *Labeling strategy to detect PLP-dependent enzymes with PLP probes.*
   Desired PLP probe is incubated with human erythrocyte lysate, incorporated into human PLP-DEs (big crescent moon = enzyme). Introduction of a fluorescent tag via click chemistry allows visualization of PLP-DEs.
**Figure 3****.** *Schematic workflow of the method of the present invention.*
   Erythrocytes are harvested from patient blood by centrifugation and treated with the probe. After introduction of the tag as seen in Figure 1 the labeled proteome is applied to a microarray coated with antibodies against PLP-DEs. Fluorescence readout allows direct determination of the patient's PLP status.
**Figure 4****.** *Method of the present invention using second anti-PLP-DE antibodies.*
   The PLP-DE enzymes in the lysate are labelled with the PLP cofactor probes and bioorthogonally attached to a label. After capturing these labelled PLP-DE enzymes, a second polyclonal antibody (that can be the same as the capturing antibody due to its polyclonality) labelled with a second label different to the PLP-probe label. Two signals can be detected, where the PLP-probe signal reflects the loading state of the cofactor and the antibody signal reflects the total amount of PLP-DE enzyme in the sample. By comparing these to signals, a ratio PLP loading state/total PLP-DE amount can be obtained.

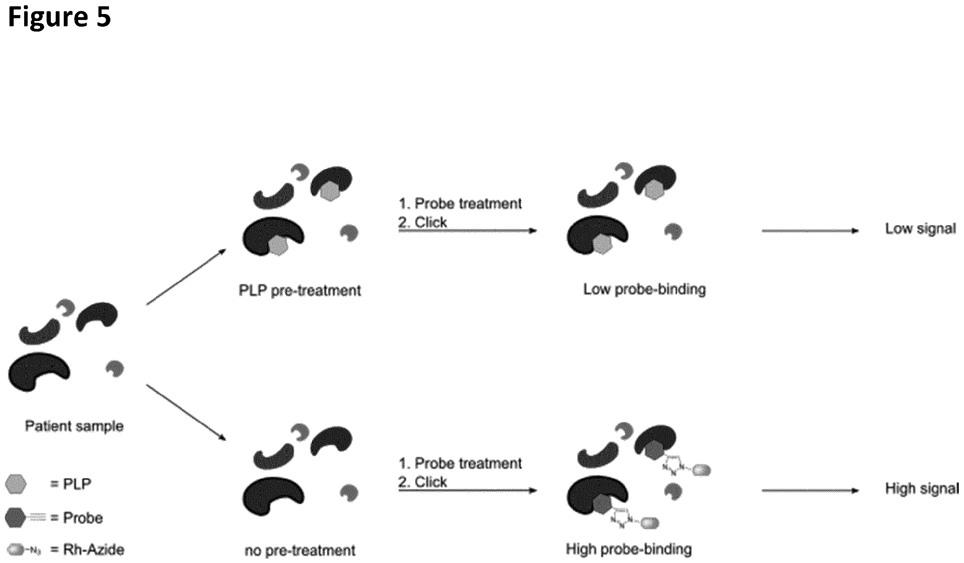

**Figure 5****.** *Schematic workflow of the method of the present invention for relative quantification of PLP status.*
   Pre-treatment of patient's sample with defined PLP standard prior to probe treatment results in lower overall fluorescent signals compared to the non-pretreated sample. Comparison of the obtained signals upon fluorescent readout on the microarray allows the determination of the patient's PLP status.

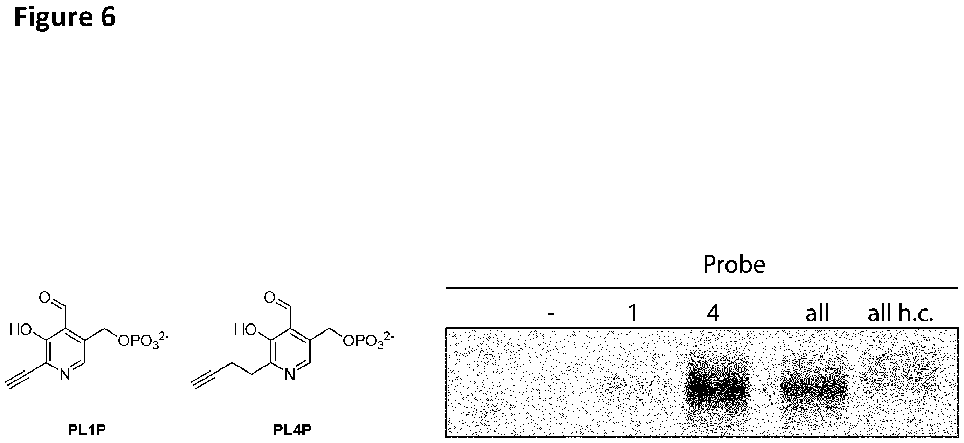

**Figure 6****.** *Recombinant labelling of human PLPBP with different PLP cofactor mimics PL1P and PL4P, and labelling with several PLxPs (all including PL1P, PL4P and PL13P). H.c.* = *heat control.*
   Conditions: 1 µM PLPBP, 100 µM probe (incubation 1 h, r.t.), reduction (NaBH₄), precipitation (Aceton), wash (MeOH), redissolve in PBS + 0.2% SDS, click to Cy5. Analysis via fluorescence SDS-Page.

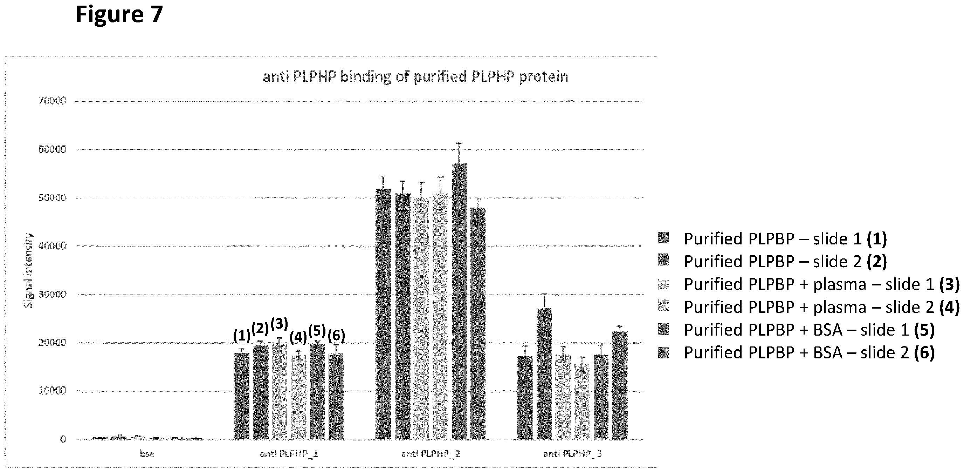

**Figure 7****.** *anti-PLPHP binding of purified PLPHP protein.*
   Signal intensities after fluorescent readout of the microarray with recombinant PLPBP samples. Sample preparation: 6 µM PLPBP (210 µL → ~ 40 µg/sample), 100 µM PL4P, 1 h, r.t, reduction (NaBH₄), precipitation (acetone), wash, redissolve in PBS, Click to Cy5, 1h r.t., precipitation (acetone), wash, redissolve 2% Tween20. **(1)** and **(2):** purified recombinant PLPBP (1), **(3)** and **(4):** purified recombinant PLPBP + plasma, **(5)** and **(6):** purified PLPBP + BSA. Samples are measured in biological duplicates.

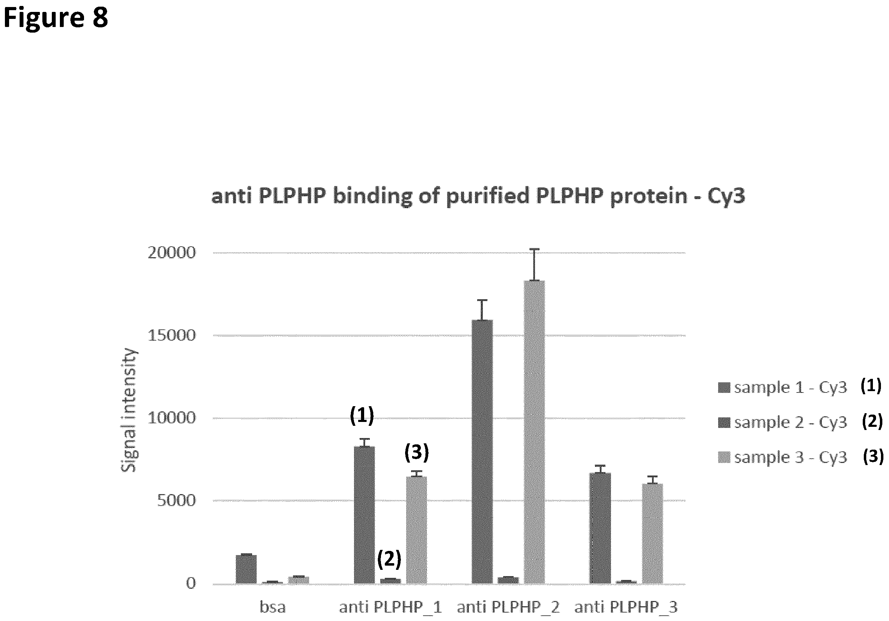

**Figure 8****.** *anti-PLPHP binding of purified PLPHP protein.*
   Signal intensities after fluorescent readout of the microarray with recombinant PLPBP samples. Facilitated sample preparation: **(1):** 6 µM PLPBP (210 µL → ~ 40 µg/sample), 100 µM PL4P, 1 h, r.t, Click to Rh-N₃, **(2):** control sample without PL4P, **(3):** control sample according to initial sample preparation, but click to Rh-N₃ instead of Cy5.

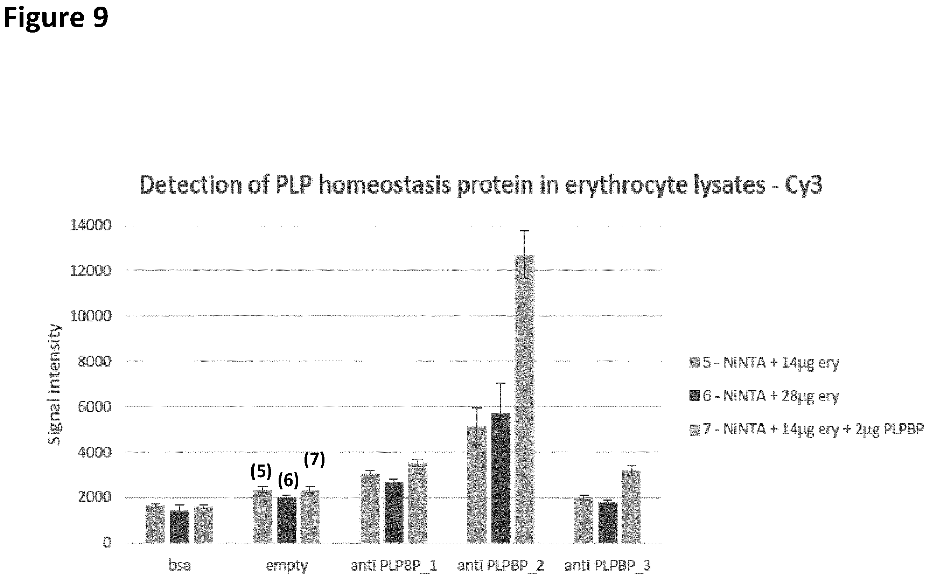

**Figure 9****.** *Detection of PLP homeostasis protein in erythrocyte lysates.*
   Signal intensities after fluorescent readout of PLPBP in erythrocyte lysate. Sample preparation: sample 5: 7 µL lysate (≈ 14 µg total protein amount after Ni-NTA), sample 6: 14 µL lysate (≈ 28 µg total protein amount after Ni-NTA), sample 7: 7 µL lysate + 2 µg PLPBP spike-in (≈ 16 µg total protein amount after Ni-NTA), Ni-NTA beads (5,7: 100 µL, 6: 200 µL), 30 min r. t., 100 µM PL4P, 1 h, r.t., Click to Rh-N₃.

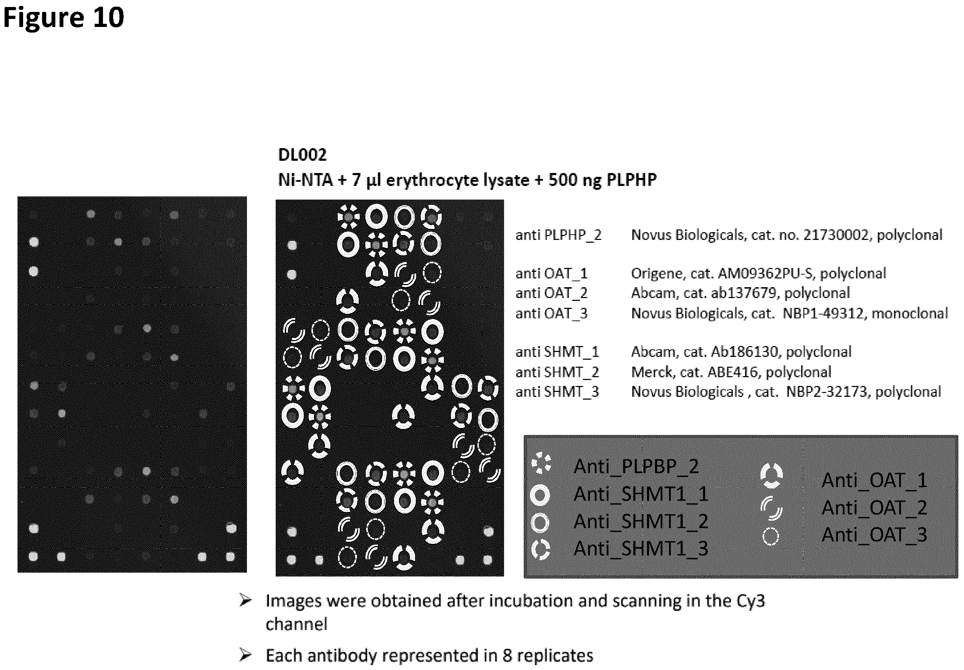

**Figure 10****.** *Example image of a microarray.*
   Example of obtained image of a microarray after incubation of sample and scanning of fluorescence intensity of the PLP-Probe signal in the Cy3 channel. Left and right image represent the same experiment. Experiment shows the signals obtained for erythrocyte lysate spiked with recombinant PLPBP. On this array 7 different antibodies targeting the PLP-DEs PLPBP, SHMT1 and OAT are immobilized in replicates. Different circles show the positions of the different antibodies on the microarray. The intense signals (lower right and left corner (3 signals) and upper left (2 signals) that are not marked with circles are positional controls to ensure a correct read-out.

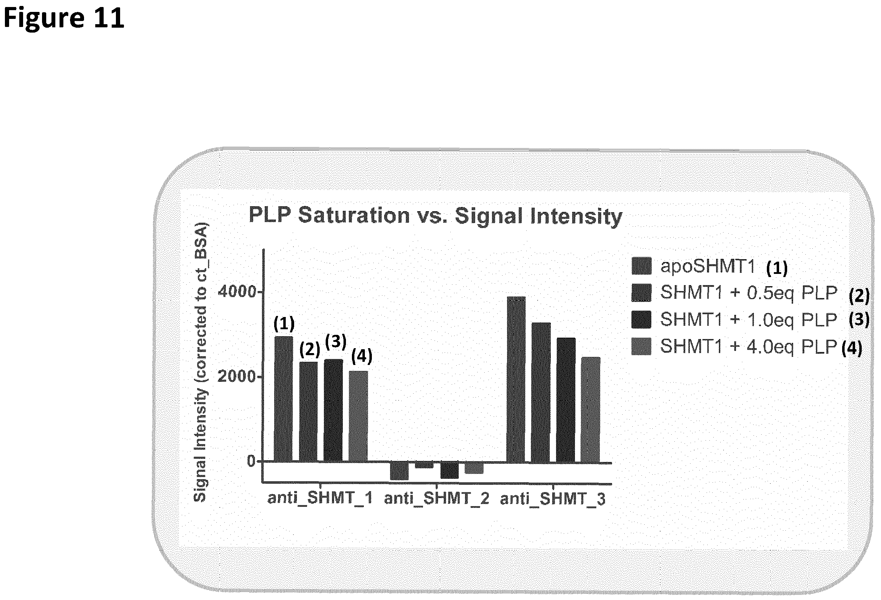

**Figure 11****.** *Signal intensity is dependent on saturation level of PLP-DE with PLP.*
   Signal intensities after fluorescent readout of recomb. SHMT1 with different PLP loading states. Sample preparation: sample **(1):** 25 µL recombinant apoSHMT1 (40 µM, ≈ 56 µg total protein amount), sample **(2):** 25 µL recomb. apoSHMT1 (40 µM, ≈ 56 µg total protein amount) + 0.5 eq. PLP (1 µL of 500 µM PLP), **(3)** 25 µL recomb. apoSHMT1 (40 µM, ≈ 56 µg total protein amount) + 1.0 eq. PLP (2 µL of 500 µM PLP), **(4)** 25 µL recomb. apoSHMT1 (40 µM, ≈ 56 µg total protein amount) + 4.0 eq. PLP (2 µL of 2 mM PLP, incubation with PLP 1h, r.t., reduction with NaBH₄ (final 20 mM) 30 min, r.t., precipitate with acetone (-80°C), centrifuge, wash and redissolve in PBS, add 100 µM PL4P, 1 h, r.t., Click to Rh-N₃.
**Figure 12****.** *Vitamin status assessment* - *Comparison via PLPBP sandwich readout.* Comparison of two healthy volunteers with differences in their vitamin B6 status (as pre-determined via HPLC) verify the suitability of the method of the present application for vitamin status assessment. Person 1 (HPLC: c(B6) = 28 ng/mL), Person 2 (HPLC: c(B6) = 14 ng/mL). The figure shows the ratio of obtained signal intensity from the probe representing the PL4P level vs. the signal intensity obtained from the secondary antibody representing the total protein amount of the sample. Low ratios indicate that less PLxP probe is bound normalized to the whole protein amount present in the sample of Person 1. This refers to a higher saturation of the PLP-DE with PLP/vitamin B6. High ratios indicate that more PLxP probe is bound normalized to the whole protein amount present in the sample of Person 2. This refers to a lower saturation of the PLP-DE with PLP/vitamin B6. These obtained results are in accordance with predetermined HPLC values of vitamin B6 status of healthy volunteers.

### EXAMPLES

### EXAMPLE 1 Materials and Methods

### 1.1 Probe Synthesis

Probes were synthesized and phosphorylation of probes was performed by S. aureus pyridoxal kinsa (SaPLK) according to the published procedures in Hoegl *et al.* (2018) and Pfanzelt et al. (2022). Angewandte Chemie (2022) https://doi.org/10.1002/anie.202117724

### 1.2 Gel-Based Labeling of PLPBP with PlxP probes

Gel-based labelling of recombinant proteins was performed as described previously (Hoegl *et al.,* 2018). In brief, PLPBP (1 µM in PBS, total volume: 50 µL) is labelled with PLxP probe (final conc. 100 µM), incubated for 1h at r.t. and reduced with NaBH₄ previous to *Click* chemistry to attach rhodamine azide. Samples were analyzed by SDS-PAGE and fluorescence scanning.

### 1.3 Protein Overexpression of PLPBP and apoSHMT1

PLPBP and SHMT1 were recombinantly expressed as described previously (Fux *et al.,* 2019). In brief, *E. coli* Rosetta2 (DE3) (Merck, Cat# 71400) carrying the expression plasmids were cultured as described and expression was induced through the addition of 0.2 µg/mL anhydrotetracycline (ATET). Bacteria were harvested and washed with PBS (6,000 × *g*, 4°C).The cell pellet was resuspended in strep binding buffer and lysed by sonication. The lysate was clarified by centrifugation (36,000 × *g*, 30 min, 4°C). Supernatant was loaded onto a StrepTrap HP column (5 mL, GE Healthcare, Cat# 28-9075) equilibrated with binding buffer using an Äkta purification system (GE Healthcare). After washing, proteins were eluted in binding buffer containing 2.5 mM desthiobiotin. For preparation of apo-SHMT1, the column was washed with 20 mL binding buffer containing 25 mM hydroxylamine prior to elution. SEC was applied for further purification. Proteins were loaded onto columns equilibrated with SEC buffer. Concentration of PLPBP and apoSHMT1 was calculated according to Lambert-Beer's law from the absorbance at 280 nm recorded in an InfiniteM200 PRO reader (TECAN, Cat# IN-MNANO). Proteins were stored at -80°C in small aliquots. Intact Protein MS is performed to check protein mass and PLP loading state of the enzyme.

### 1.4 Intact Protein MS

IP-MS measurements were perfomed on an Ultimate 3000 RSLC system (Thermo Scientific) coupled to a LTQ Orbitrap XL mass spectrometer (Thermo Scientific). Protein desalting was carried out using a MassPREP desalting column (Waters) at 25 °C. Gradient elution was carried out with 0.1% formic acid (LC-MS grade, Fisher Analytics) in water (LC-MS grade, Fisher Analytics) (A) and 0.1% formic acid in acetonitrile (MeCN, LC-MS grade, Fisher Analytics) (B). After 2 min pre-equilibration with 6% B, protein samples were injected and eluted with a linear gradient from 6% to 95% B over 1.5 min and 2 min at 95% B at 300 µL/min flow rate. The column was re-equilibrated with 6% B for 1 min. Mass spectrometric measurements were conducted in HESI positive mode (H-ESI-II source, Thermo Scientific) with the following parameters: 4.0 kV capillary voltage, 350 °C capillary temperature, 31 V capillary voltage, 110 V tube lens, 30 L/h sheath gas, 15 L/h aux gas. Full scan measurements were accomplished in a range from 300-2000 m/z in profile mode in the orbitrap at a resolution of 100,000. Raw spectra were processed with *UniDec* 2.6.7 for deconvolution.

### 1.5 Preparation of human erythrocyte lysate

Human erythrocytes were obtained from freshly drawn whole blood in EDTA tubes (4 ml each) *via* centrifugation (1 × 3500 rpm, 6 min, 1 × 3500 rpm, 8 min, 1 × 3000 rpm, 8 min). Supernatant (blood plasma) and buffy coat is removed and washed with PBS (5 mL) after each centrifugation step. To 3-4 mL erythrocytes are added 3-4 mL lysis buffer (Tris 50 mM, NaCl 150 mM, 1% NP40 (v/v), pH 7.5), incubated on ice for 5 min and frozen at -80°C o/n. Thawed erythrocyte lysate is centrifuged (13 000 rpm, 10 min, 4 °C) and total protein amount is determined by BCA Assay (ROTI^{®}Quant, Roth). Erythrocyte lysate is stored in small aliquots at -80°C.

### 1.6 Sample Preparation for Microarray Analysis - Ni-NTA Depletion

Ni-NTA agarose beads (Qiagen) are carefully resuspended by inversion on an Eppendorf tube wheel at 4 °C and transferred to an Eppendorf tube (X × 100 µL + 25% in one LoBind Eppendorf tube with marked filling level before washing steps are performed. The beads are washed with PBS (3 × 1 mL), centrifuged (400 g, 3 min, r.t.) and filled to the original level with PBS. To 100 µL of bead suspension are added 7 µL erythrocyte lysate and incubated for 30 min at r.t.. The samples are transferred quantitatively onto biospin columns and centrifuged (1000 g, 2 min, r.t.). Collected flow through is filled up to a final volume of 100 µL with PBS and further subjected to probe labelling.

### - Labelling with PLxP probe

Recombinant protein or erythrocyte lysate in PBS (total volume: 100 µL) are incubated with 40 µM PlxP probe (addition of 1-2 µL of respective stock) for 1h at room temperature. Samples are either directly subjected to *Click* chemistry or if irreversible probe biding is required to reduction with NaBH₄.

### - Optional: Reduction with NaBH₄

To irreversible bind the respective PlxP probe to PLP-DE, the internal aldimine is reduced to the respective secondary amine with NaBH₄.

Labelled recombinant protein or erythrocyte lysate in PBS are treated with 20 mM NaBH₄ (250 mM stock solution in 0.1 M NaOH) and incubated for 30 min at r.t. Reduction is quenched by adding cold acetone (4 × volume of sample, -20°C) for at least 1h or o/n. Precipitated protein is pelletized (10 000 rpm, 15 min, 4 °C) and supernatant is discarded. For washing, protein pellet is resuspended in 0.5 mL cold methanol (-80 °C) by mild sonication (10 s, 10% intensity) and centrifuged (10 000 rpm, 10 min, 4 °C). Finally, each protein pellet is resuspended in 100 µL PBS by sonication (10 s, 10% intensity).

### - Click Chemistry Click conditions: 100 µM Rh-N₃, 100 µM TBTA, 1 mM TCEP, 1 mM CuSO₄

The samples are subjected to *Click* reaction by adding 1 µL rhodamine azide (10 mM in DMSO), 6 µL TBTA (1.667 mM in *t*BuOH/DMSO 80:20), 1 µL TCEP (15 mg/mL in ddH₂O) and 2 µL CuSO₄ (50 mM in ddH₂O) to each sample (100 µL) and incubated for at least 1 h at r.t. Samples are subsequently analysed on the microarrays.

### - Optional: Removal of excess dye prior to incubation

For the first experiments (Figure 7), excess rhodamine dye is removed prior to incubation on the microarrays. For this, *Click* reaction is quenched by adding cold acetone (4 × volume of sample, -20°C) for at least 1h or o/n. Precipitated protein is pelletized (10 000 rpm, 15 min, 4 °C) and supernatant is discarded. For washing, protein pellet is resuspended in 0.5 mL cold methanol (-80 °C) by mild sonication (10 s, 10% intensity) and centrifuged (10 000 rpm, 10 min, 4 °C). Finally, each protein pellet is resuspended in PBS (100 µL).

### 1.7 Microarray Production

Custom microarrays were produced using the automated production facility at Sciomics. Standard production protocols using contact printing on chemically modified glass surfaces (Epoxy-coupling groups) were used and slides were individually quality controlled after production. Produced Slides were stored according to Sciomics SOPs cold and protected from light.

### 1.8 Antibody Labelling

The secondary antibodies were labelled at an adjusted protein concentration for two hours with scioDye2 (Sciomics, Germany). After two hours the reaction was stopped and the buffer exchanged to PBS. All labelled protein samples were stored at -20° C until use.

### 1.9 Samples and protein extraction

The samples were used immediately after performing Click chemistry without any freeze/thaw cycles and only minimal storage times at 4°C.

### 1.10 Sample incubation

The samples were analysed in a single colour approach (without secondary antibody incubation) or dual-colour approach (with secondary antibody incubation) using custom microarrays containing antibodies against OAT, SHMT and PLPBP or a subset of these antibodies. Each antibody is represented on the array in 16 replicates. The arrays were blocked with scioBlock (Sciomics) on a Hybstation 4800 (Tecan, Austria) and afterwards the samples were incubated. After incubation for three hours, the slides were thoroughly washed with 1x PBSTT, rinsed with 0.1x PBS as well as with water and subsequently dried with nitrogen.

### 1.11 Data acquisition and analysis

Slide scanning was conducted using a Powerscanner (Tecan, Austria) with constant instrument laser power and PMT settings. Spot segmentation was performed with GenePix Pro 6.0 (Molecular Devices, Union City, CA, USA). Acquired raw data were analysed using Microsoft Excel for all calculations such as average signal values and standard deviations.

### EXAMPLE 2 Results

### 2.1 Experiments for setup of the B6VitaStat microarray

### a) Recombinant labelling of human PLPBP with different functionalized PLP cofactors.

Conditions: 1 µM PLPBP, 100 µM probe (incubation 1 h, r.t.), reduction (NaBH₄), precipitation (aceton), wash (MeOH), redissolve in PBS + 0.2% SDS, click to Cy5. Analysis via fluorescence SDS-Page. Results see Figure 6.

Human PLP binding protein (PLPBP, UniProt O94903) was used as a model PLP-DE in human erythrocytes. The following two PLP cofactor mimics were successfully tested: PL1P, PL4P, wherein PL4P was identified as the most suitable PLP cofactor mimic for labeling of recombinant PLPBP *via* SDS-Page.

### b) anti-PLPBP antibodies

To further validate the method of the invention, three different anti-PLPBP antibodies tested:
anti PLPHP_1: Thermofischer, cat. nr. PA532036, polyclonal
anti PLPHP_2: Novusbio, cat. nr. 21730002, polyclonal
anti PLPHP_3: Origene, cat. nr. TA505130, monoclonal

The antibodies were immobilized on glass sides, which contain several replicates of the antibody for quality control and statistical evaluation. Recombinant PLPBP incubated with PL4P and clicked to Cy5 was used. Results see Figure 7 showing that all three immobilized antibodies against PLPBP work.

### c) In general, overall concept and microarray works with good S/N ratios. Plasma and BSA do not interfere with the workflow and fluorescent analysis.

The experimental conditions were further optimized to a sample preparation, where no reduction of the internal aldimine to the secondary amine, no precipitation and removal of excess dye is necessary prior to the incubation on the microarray with subsequent fluorescent analysis. Additionally, negative (sample 2) and positive (sample 3) controls verify the overall concept for recombinant PLPBP detection on our microarray with immobilized antibodies against PLPBP. Results see Figure 7 and 8.

### d) Signal intensity dependent on PLP saturation of enzyme.

To further validate the method of invention, it was proven that the obtained fluorescent signal is dependent on the PLP saturation state of the enzyme. Hereby, serine hydroxymethyltransferase 1 was recombinantly expressed as the apo-enzyme and gradually saturated with different amounts of PLP, that was irreversibly bound to the SHMT1 enzyme *via* NaBH₄ to ensure a fixed saturation state (verified by IP-MS). The observed decrease in signal intensity with increasing PLP saturation shown in Figure 11 is in accordance with our inverse correlation theory that less PL4P probe can bind to a higher PLP saturated enzyme.

### 2.2 Experiments with human erythrocyte lysate

With the results of 2.1, we started our test procedures in human erythrocyte lysate.

Initial experiments revealed the inference of hemoglobin with the microarray. According to literature, hemoglobin and carboanhydrase-1 account for up to 98% of the total soluble protein amount in erythrocytes that are often hindering the analysis of blood containing samples and identification of less abundant proteins like PLP-Des (Ringrose *et al.,* 2008). We adapted a literature procedure by Ringrose *et al.* (2008) and Yang *et al.* (2012) and depleted hemoglobin in our erythrocyte lysate with Ni-NTA beads prior to incubation with our PL4P probe. The additional background reduction step enabled the successful detection of endogenous PLPBP in human erythrocytes with our PL4P probe proving our overall B6VitaStat concept. Spike-in of recombinant PLPBP to the human erythrocyte lysate additionally shows a dependency of the signal intensity on the amount of PLP-DE present in the sample. Results see Figure 9. This is also visible in the obtained microarray images (see Figure 10), where human erthyrocyte lysate was also spiked with PLPBP. This image additionally shows the extension of our microarray format to several PLP-DEs. In total, 7 different antibodies targeting the three PLP-DEs PLPBP, SHMT1 and OAT showed very good to moderate signal intensities proving that the array can be exploited for all PLP-DEs if suitable antibodies are available.

Lastly, due to the proven dependency of the fluorescence intensity of the amount of protein present in the sample and the saturation state of the enzyme, the microarray is extended to a sandwich format to enable normalization of signal intensities and to interpret the vitamin status of a subject. Therefore, after incubation of the PLP treated lysate on the microarrays, a second antibody bearing a different fluorescent label than the PLP probe is added and a two-channel fluorescence readout is performed. Herein, high ratios of PLP probe/protein amount would indicate low vitamin B6 status. Comparison of two healthy volunteers with huge differences in their vitamin B6 status (determined via HPLC) verified this theory making this platform a novel platform for vitamin status assessment (see Figure 12).

The features disclosed in the foregoing description, in the claims and/or in the accompanying drawings may, both separately and in any combination thereof, be material for realizing the invention in diverse forms thereof.

The project leading to this application has received funding from the European Research Council (ERC) under the European Union's Horizon 2020 research and innovation program (grant agreement No. 875594).

### REFERENCES

Brussaard, J., Löwik, M., Van den Berg, H., Brants, H. & Bemelmans, W. Dietary and other determinants of vitamin B6 parameters. European journal of clinical nutrition 51, S39-45 (1997).

Bryk, A. H. & Wiśniewski, J. R. Quantitative analysis of human red blood cell proteome. Journal of proteome research 16, 2752-2761 (2017).

CDC. Second National Report on Biochemical Indicators of Diet and Nutrition in the U.S. Population. 495 (Center for Disease Control and Prevention, 2012).

Dalery, K. et al. Homocysteine and coronary artery disease in French Canadian subjects: relation with vitamins B12, B6, pyridoxal phosphate, and folate. The American journal of cardiology 75, 1107-1111 (1995).

Fux, A., Pfanzelt, M., Kirsch, V. C., Hoegl, A. & Sieber, S. A. Customizing Functionalized Cofactor Mimics to Study the Human Pyridoxal 5'-Phosphate-Binding Proteome. Cell Chemical Biology 26, 1461-1468.e1467, doi:https://doi.org/10.1016/j.chembiol.2019.08.003 (2019).

Gregory, J. & Kirk, J. Vitamin B6 in foods: assessment of stability and bioavailability. (1977).

Hoegl, A. et al. Mining the cellular inventory of pyridoxal phosphate-dependent enzymes with functionalized cofactor mimics. Nature chemistry 10, 1234-1245 (2018).

Huang, Y.-C., Chen, W., Evans, M. A., Mitchell, M. E. & Shultz, T. D. Vitamin B-6 requirement and status assessment of young women fed a high-protein diet with various levels of vitamin B-6. The American journal of clinical nutrition 67, 208-220 (1998).

Hustad, S. et al. Kinetic modeling of storage effects on biomarkers related to B vitamin status and one-carbon metabolism. Clinical chemistry 58, 402-410 (2012).

Johansson, M. et al. Serum B vitamin levels and risk of lung cancer. Jama 303, 2377-2385 (2010).

Kanchi, S., Sabela, M. I., Mdluli, P. S., Inamuddin & Bisetty, K. Smartphone based bioanalytical and diagnosis applications: A review. Biosensors and Bioelectronics 102, 136-149, doi:https://doi.org/10.1016/j.bios.2017.11.021 (2018).

Kelly, P. et al. Inflammation, homocysteine, and vitamin B6 status after ischemic stroke. Stroke 35, 12-15 (2004).

Leklem, J. E. Vitamin B-6: a status report. The Journal of nutrition 120, 1503-1507 (1990).

Lotto, V., Choi, S.-W. & Friso, S. Vitamin B6: a challenging link between nutrition and inflammation in CVD. British journal of nutrition 106, 183-195 (2011).

Panton, K. K., Farup, P. G., Sagen, E., Sirum, U. F. & Åsberg, A. Vitamin B6 in plasma-sample stability and the reference limits. Scandinavian journal of clinical and laboratory investigation 73, 476-479 (2013).

Pfanzelt, M., Maher, T.E., Absmeier, R.M., Schwarz, M., Sieber, S.A. Tailored Pyridoxal Probes Unravel Novel Cofactor-Dependent Targets and Antibiotic Hits in Critical Bacterial Pathogens. Angewandte Chemie (2022) https://doi.org/10.1002/anie.202117724

Ringrose, J. H. et al. Highly Efficient Depletion Strategy for the Two Most Abundant Erythrocyte Soluble Proteins Improves Proteome Coverage Dramatically. Journal of Proteome Research 7, 3060-3063, doi: 10.1021/pr8001029 (2008).

Reynolds, R. D. in Vitamin B-6 Metabolism in Pregnancy, Lactation, and Infancy 41-59 (CRC Press, 1995).

Rimm, E. B. et al. Folate and vitamin B6 from diet and supplements in relation to risk of coronary heart disease among women. Jama 279, 359-364 (1998).

Robinson, K. et al. Low circulating folate and vitamin B6 concentrations: risk factors for stroke, peripheral vascular disease, and coronary artery disease. Circulation 97, 437-443 (1998).

Sies, H., Stahl, W. & Sundquist, A. R. Antioxidant functions of vitamins: Vitamins E and C, Beta-Carotene, and other carotenoids a. Annals of the New York Academy of Sciences 669, 7-20 (1992).

Ueland, P. M., Ulvik, A., Rios-Avila, L., Midttun, Ø. & Gregory, J. F. Direct and functional biomarkers of vitamin B6 status. Annual review of nutrition 35, 33-70 (2015).

Van der Ham, M. et al. Quantification of vitamin B6 vitamers in human cerebrospinal fluid by ultra performance liquid chromatography-tandem mass spectrometry. Analytica chimica acta 712, 108-114 (2012).

Vanuzzo, D. et al. Both vitamin B6 and total homocysteine plasma levels predict long-term atherothrombotic events in healthy subjects. European heart journal 28, 484-491 (2007).

Verhoef, P. et al. Homocysteine metabolism and risk of myocardial infarction: relation with vitamins B6, B12, and folate. American journal of epidemiology 143, 845-859 (1996).

Vermaak, W. et al. Vitamin B-6 nutrition status and cigarette smoking. The American journal of clinical nutrition 51, 1058-1061 (1990).

Yang, H., Zhou, B., Prinz, M. & Siegel, D. Proteomic Analysis of Menstrual Blood∗. Molecular & Cellular Proteomics 11, 1024-1035, doi:https://doi.org/10.1 12.018390 (2012).

## Claims

1. An *in vitro* method for determining the vitamin status, in particular vitamin B6, B2, B 12 and/or D3, of a subject comprising
(a) harvesting erythrocytes from a sample obtained from a subject or providing erythrocytes obtained from a sample of a subject;
(b) providing a probe comprising at least one functionalized cofactor of PLP-dependent enzymes (PLP-DEs) (PLP probe);
(c) lysing the erythrocytes and thereby releasing the proteome including the PLP-DEs and then treating the lysate with the PLP probe provided in (b); or
(c') treating the erythrocytes with the PLP probe provided in (b) and then lysing the erythrocytes and thereby releasing the proteome including the PLP-DEs;
(d) adding a label for the PLP-probe to the treated lysate of (c) or to the lysate of (c');
(e) capturing the labeled PLP-DEs with anti-PLP-DE-antibodies immobilized on a surface, preferably an array or a membrane,
(f) detecting the labeled PLP-DEs, and
(g) determining the vitamin status of the subject.

2. The method of claim 1, wherein in step (a) the erythrocytes are harvested by centrifugation, optionally including washing step(s), and the sample is whole blood.

3. The method of claim 1 or 2, wherein the cofactor of PLP-DEs is functionalized with a biorthogonal labeling group,
wherein preferably the biorthogonal labeling group is selected from an alkyne or azide moiety or a tetrazine functionality,
wherein the cofactor of PLP-DEs is optionally further functionalized with a phosphoamidate.

4. The method of claim 3, wherein the biorthogonal labeling group comprises an alkyne group,
and the cofactor probe is preferably selected from PL1P, PL2P, PL4P to PL13P,
or wherein the biorthogonal labeling group is an azide group, and the cofactor probe is preferably PL3P.

5. The method of any one of claims 1 to 4, wherein the probe comprises at least two functionalized cofactors of PLP-DEs or it comprises three or more functionalized cofactors of PLP-DEs,
and/or wherein the probe comprises a further moiety or tag, such as biotin.

6. The method of any one of claims 1 to 5, wherein the lysis of the erythrocytes in step (c) or in step (c') comprises the removal of hemoglobin.

7. The method of any one of claims 1 to 6, wherein the treatment in step (c) comprises an incubation of the lysate with the PLP probe, and optional addition of a reducing agent,
or wherein the treatment in step (c') comprises an incubation of the erythrocytes with the PLP probe.

8. The method of any one of claims 1 to 7, wherein the label in step (d) is
a fluorescent label, such as rhodamines, cyanine dyes (Cy3, Cy5), fluoresceines,
a bioluminescence label,
a chemiluminescence label,
a dye,
or combinations thereof
wherein the label is attached to the biorthogonal labeling group of the cofactors,
such as via click chemistry or Staudinger ligation.

9. The method of any one of claims 1 to 8, wherein in step (e) second anti-PLP-DE-antibodies are added, wherein said second anti-PLP-DE-antibodies are labelled,
wherein the label of said second anti-PLP-DE-antibodies is different to the label of step (d) and is selected from a fluorescent label, a bioluminescence label, a chemiluminescence label, a dye, or combinations thereof;
wherein in step (f) the label of said second anti-PLP-DE-antibodies is detected in addition to the signal of the labeled PLP-DEs and a ratio of PLP loading state/total PLP-DE amount is obtained by comparing the signal of the labeled PLP-DEs and the signal of the second antibodies.

10. The method of any one of claims 1 to 9, wherein in step (g) the vitamin status, preferably the vitamin B6 status, is determined by comparing the readout of step (f), such as fluorescence intensity, bioluminescence intensity, or absorbance, with a control, such as recombinant PLP-DEs with defined cofactor loading or reference samples where the PLP status is known,
and/or wherein the method is a point of care method.

11. The method of any one of claims 1 to 10, further comprising
dividing the sample obtained from the subject in two parts and pre-treating one part with a PLP standard prior to step (c),
performing steps (a) to (f) for both parts in parallel,
comparing the results of step (f) for both parts and thereby quantifying the PLP status.

12. The method of any one of claims 1 to 11, wherein the vitamin status determined in a subject is used for:
- determining the vitamin B6 status of the subject,
- monitoring the PLP status and/or vitamin B6 status of the subject,
- diagnosing vitamin B6-deficiency and vitamin B6-deficiency-associated diseases,
in particular related to age, malnutrition, and/or alcoholism.
in dialysis patients,
inflammatory bowel disease.

13. A kit for *in vitro* determining the vitamin status, in particular vitamin B6, B2, B12 and/or D3, in a sample of a subject, comprising
(1) a probe comprising at least one functionalized cofactor of PLP-dependent enzymes (PLP-DEs) (PLP probe), said probe being preferably as defined in any one of claims 3 to 5;
(2) a label for the PLP-probe, which is preferably as defined in claim 8;
(3) optionally, an array for PLP-DEs, preferably comprising anti-PLP-DE-antibodies,
(4) optionally, reagents for lysing erythrocytes, reducing agents, and control(s).

14. An immunographic device for *in vitro* determining the vitamin status, in particular vitamin B6, B2, B12 and/or D3, in a sample of a subject,
said device comprising a solid carrier or surface coated with anti-PLP-DE-antibodies or with nanoparticles comprising a moiety or tag which binds to a probe comprising at least one functionalized cofactor of PLP-dependent enzymes (PLP-DEs) (PLP probe),
wherein the immunographic device is preferably a lateral flow assay (LFA) device, more preferably a point-of-care device.

15. The immunographic device of claim 14, wherein the immunographic device comprises a porous membrane operably connected to
(a) a portion or pad, where a processed sample is applied,
(b) a test portion or test line comprising said anti-PLP-DE-antibodies or said nanoparticles, preferably gold nanoparticles,
(c) a control portion or control line; and
(d) an absorbent portion/pad,
wherein, preferably, the sample is whole blood or erythrocytes.
wherein, when the sample is more preferably whole blood, the erythrocytes are preferably harvested therefrom,
and/or wherein, preferably, obtaining a processed sample comprises:
the erythrocytes or the harvested erythrocytes are lysed and thereby release the proteome including the PLP-dependent enzymes (PLP-DEs),
the lysate is treated with a probe comprising at least one functionalized cofactor of PLP-dependent enzymes (PLP-DEs) (PLP probe), said probe being as defined in any one of claims 3 to 5, and
a label for the PLP-probe, which is as defined in claim 8, is added to the treated lysate, and
the processed sample is obtained,
or wherein, preferably, obtaining a processed sample comprises:
the erythrocytes or the harvested erythrocytes are treated with a probe comprising at least one functionalized cofactor of PLP-dependent enzymes (PLP-DEs) (PLP probe), said probe being as defined in any one of claims 3 to 5, preferably a cofactor of PLP-DEs which is functionalized with a phosphoamidate,
the treated erythrocytes are then lysed and thereby release the proteome including the PLP-dependent enzymes (PLP-DEs),
a label for the PLP-probe, which is as defined herein above, is added to the lysate, and
the processed sample is obtained.
